# EUROPEAN PATENT APPLICATION

(11) **EP 3 818 976 A1**
(43) Date of publication of application: **12.05.2021**
(21) Application number: 19831440.3
(22) Date of filing: 10.04.2019
(51) Int. Cl.: A61K 9/70, A61K 31/137, A61K 45/00, A61K 47/59, A61L 15/26, A61L 15/42, A61P 9/08, A61P 25/04, A61P 29/00, A61P 29/02, A61P 31/04, A61P 31/10

(54) **STRUCTURE**

(30) Priority: 02.07.2018 JP 2018126245; 20.09.2018 JP 2018175830
(71) Applicant: NITTO DENKO CORPORATION, Ibaraki-shi Osaka 567-8680 (JP)
(72) Inventor: MAENO Yohei, Ibaraki-shi, Osaka 567-8680 (JP)
(74) Representative: Müller-Boré & Partner Patentanwälte PartG mbB
(86) International application number: PCT/JP2019/015653
(87) International publication number: WO 2020/008695

(57) **Abstract**

The present invention provides a structure suitable for controlling the release quantity of an active ingredient in a state where the structure is in contact with skin. A structure 10 according to the present invention includes a retention part 1 that retains an active ingredient, and a release control layer 3 that controls release of the active ingredient to an outside. In the structure 10, at least one of the following conditions holds: i) the release control layer 3 includes a microporous membrane having an average pore diameter of 0.01 µm or less; and ii) the release control layer 3 is a nanofiltration membrane or a reverse osmotic membrane.

## Description

### TECHNICAL FIELD

The present invention relates to a structure suitable for releasing an active ingredient such as a drug and typified by a transdermal absorption preparation.

### BACKGROUND ART

Structures that gradually release an active ingredient have been put in practical use as transdermal absorption preparations. The transdermal absorption preparations are characterized in that they make it possible to avoid a first-pass effect caused by metabolic turnover of a drug in the liver when the drug is absorbed from a digestive organ, and thus can maintain stably the concentration of the drug in blood for a long time. The transdermal absorption preparations are also a noninvasive pharmaceutical form that simplifies drug administration.

The transdermal absorption preparations are roughly divided into reservoir type and matrix type. The reservoir type transdermal absorption preparations include a retention part that retains a drug, and a release control membrane through which the drug to be released from the retention part passes. The reservoir type transdermal absorption preparations are usually applied to skin via an adhesive layer disposed on the release control membrane side. In contrast, the matrix type transdermal absorption preparations have no release control membrane. The matrix type transdermal absorption preparations usually include a support body and an adhesive layer containing a drug.

Patent Literature 1 discloses general configurations and members of the reservoir type and matrix type transdermal absorption preparations. Patent Literature 1 discloses a release control membrane that is a microporous membrane having an average pore diameter of 0.1 µm to 1 µm. Patent Literature 1 mentions polyolefin and polytetrafluoroethylene as examples of the material of the microporous membrane.

A common technical challenge shared by these transdermal absorption preparations is to improve the transdermal absorbability of a drug. As mentioned in Patent Literature 1, it is suggested to use, together with a drug, various kinds of absorption enhancers in order to improve the transdermal absorbability of a drug.

### CITATION LIST

### Patent Literature

Patent Literature 1: JP 2014-172885 A

### SUMMARY OF INVENTION

### Technical Problem

Structures practically used as transdermal absorption preparations are designed so as to always release an active ingredient in a state where the structures are in contact with skin. This makes it impossible to stop or reduce the release of the active ingredient as appropriate in a state where such a structure is in contact with skin. Thus, in order to intermittently absorb the active ingredient over a long period of time, it is necessary to separate the structure from skin as needed to stop the release of the active ingredient, and then bring the structure into contact with the skin again to resume the absorption of the active ingredient, or it is necessary to prepare a plurality of structures each containing a small quantity of the active ingredient and use them one after another by replacing properly. This turns out not only to be complicated for users but also to increase the burden of costs. No attention has been paid to this problem so far, but solving it will lead to enhanced convenience of the structures, and thus broader applications of the structures can be expected as well.

An object of the present invention is to provide a structure suitable for controlling the release quantity of an active ingredient in a state where the structure is in contact with skin.

### Solution to Problem

From a first aspect, the present invention provides
a structure including a retention part that retains an active ingredient, and a release control layer that controls release of the active ingredient to an outside, wherein
at least one of the following conditions holds:
i) the release control layer includes a microporous membrane having an average pore diameter of 0.01 µm or less; and
ii) the release control layer is a nanofiltration membrane or a reverse osmotic membrane.

From a second aspect, the present invention provides
a structure including a retention part that retains an active ingredient, and a release control layer that controls release of the active ingredient to an outside, wherein
the release control layer has a molecular weight cutoff of 20000 or less.

From a third aspect, the present invention provides
a structure including a retention part that retains an active ingredient, and a release control layer that controls release of the active ingredient to an outside, wherein
a releasing speed at which the active ingredient is released per area of 1 cm² of a principal surface of the release control layer is less than 0.39 µg/hour in a state where no pressure is applied to the retention part from the outside, and
the releasing speed at which the active ingredient is released per area of 1 cm² of the principal surface of the release control layer is 0.39 µg/hour or more in a state where a pressure is applied to the retention part from the outside in such a manner as to transmit a pressure of 0.15 MPa to the principal surface of the release control layer.

### Advantageous Effects of Invention

The present invention can provide a structure suitable for controlling the release quantity of an active ingredient in a state where the structure is in contact with skin.

### BRIEF DESCRIPTION OF DRAWINGS

FIG. 1 is a cross-sectional view illustrating a configuration of a structure according to one embodiment of the present invention.
FIG. 2 is a schematic cross-sectional view illustrating an example of a measuring device for measuring the quantity of an active ingredient passing through a membrane corresponding to a release control layer.

### DESCRIPTION OF EMBODIMENTS

In one embodiment of the present invention, the release control layer is a nanofiltration membrane.

In one embodiment of the present invention, the nanofiltration membrane includes a dense layer, and the dense layer is made of modified polyethersulfone.

In one embodiment of the present invention, the retention part includes a support layer, and a retention chamber formed between the support layer and the release control layer.

In one embodiment of the present invention, the active ingredient contains a compound having at least one effect selected from the group consisting of an antifungal effect, an antibacterial effect, an antiinflammatory effect, an analgesic effect, and a vasorelaxant effect.

In one embodiment of the present invention, the structure further includes an adhesive layer disposed on a principal surface of the release control layer.

Hereinafter, the present invention will be described in detail. The following description, however, is not intended to limit the present invention to specific embodiments.

### (Configuration of structure)

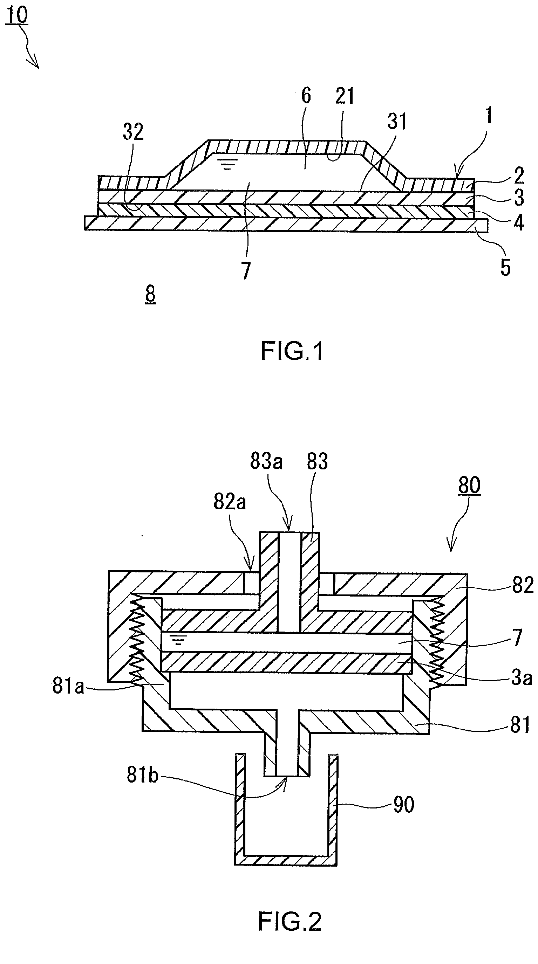

As shown in FIG. 1, a structure 10 of the present embodiment includes a retention part 1 that retains an active ingredient. The structure 10 further includes a release control layer 3 disposed between an outside 8 of the structure 10 and the retention part 1. The active ingredient to be released from the retention part 1 passes through the release control layer 3. The release control layer 3 controls the release of the active ingredient to the outside 8. The structure 10 is a laminate of the retention part 1 and the release control layer 3, for example. The retention part 1 includes a support layer 2, and a retention chamber 6 formed between the support layer 2 and the release control layer 3. The retention chamber 6 is a space formed between a principal surface 21, on the release control layer side, of the support layer 2 and a principal surface 31, on the support layer side, of the release control layer 3. In the embodiment shown in FIG. 1, the support layer 2 and the release control layer 3 have the respective principal surfaces 21 and 31 joined to each other around the retention chamber 6 so as to surround the retention chamber 6. As shown in FIG. 1, the support layer 2 may be joined, in a deformed shape, to the release control layer 3 so that the retention chamber 6 with a desired internal volume is secured between the release control layer 3 and the support layer 2. The support layer 2 and the release control layer 3 may be integrated in a state of surrounding the retention chamber 6.

In the retention chamber 6, the active ingredient is retained in the form, for example, of a composite containing the active ingredient, and more specifically, in the form of a liquid or the like containing the active ingredient. In the embodiment shown in FIG. 1, a liquid 7 containing the active ingredient is retained in the retention chamber 6. The retention chamber 6 has an internal capacity that is variable, and it is possible to reduce the internal capacity from its maximum value, that is, its internal volume, by applying a pressure from the outside. The release of the active ingredient from the retention part 1 can be accelerated by reducing the capacity inside the retention chamber 6. In other words, the pressure applied so as to reduce the internal capacity of the retention chamber 6 can be a driving force to accelerate the release of the active ingredient. The release control layer 3 serves the role of controlling appropriately the quantity of the active ingredient passing therethrough so as not to allow the release quantity of the active ingredient to be excessive in a state where no such a driving force is applied. According to the studies made by the present inventor, a microporous membrane (see Patent Literature 1) with an average pore diameter of about 0.1 µm to 1 µm is too large in terms of the quantity of an active ingredient passing therethrough to serve this role appropriately in a state without the above-mentioned driving force.

The active ingredient passing through the release control layer 3 may be accompanied by another component of the composite containing the active ingredient, instead of being present alone. Particularly, in the case where the active ingredient is retained in the retention part 1 as a liquid containing the active ingredient, the active ingredient usually passes through the release control layer 3 together with a liquid component that is typically a solvent. Thus, in this case, it is possible to calculate the release quantity of the active ingredient by evaluating the quantity of the liquid passing through the release control layer 3 and further evaluating the concentration of the active ingredient in the liquid passing through the release control layer 3.

The retention part 1 may include a layer other than the support layer 2. Examples of such an additional layer include a spacer disposed between the support layer 2 and the release control layer 3. The addition of the spacer is effective, for example, in increasing the internal volume of the retention chamber 6 and alleviating the deformation of the support layer 2. The spacer may be, for example, a film from which a portion corresponding to the retention chamber 6 has been removed.

The retention part 1 may include a retainer that is disposed in the retention chamber 6 and that retains the active ingredient. Examples of the retainer include a nonwoven fabric impregnated with a solution containing the active ingredient. The retainer may be disposed instead of the retention chamber 6. In this case, the structure 10 has, for example, a configuration obtained by stacking the release control layer 3, the nonwoven fabric constituting the retainer, and the support layer 2 in this order.

The structure 10 may further include an adhesive layer 4 and a separation film 5. The adhesive layer 4 is disposed on a principal surface 32, on the side opposite to the support layer, of the release control layer 3. In the case where the adhesive layer 4 is disposed beforehand, it becomes easy to apply the structure 10 to an object (human skin, for example). The adhesive layer 4 may cover the principal surface 32 of the release control layer 3 entirely or partially. The separation film 5 is attached to the adhesive layer 4. Removing the separation film 5 allows the adhesive layer 4 to be exposed to the outside, so that the structure can be applied to the object.

The joining of the support layer 2 to the release control layer 3 as well as the joining of the above-mentioned spacer to the layers 2 and 3 can be carried out by, for example, bonding using a glue, and thermal welding and other welding techniques. These joinings may be carried out using a double-sided adhesive tape.

### (Support layer)

The support layer 2 is preferably a layer that does not allow the active ingredient to pass therethrough. The support layer 2 may be a single layer membrane, and may be a multiple layer membrane. In the case where the support layer 2 is a single layer membrane, the support layer 2 is preferably a nonporous membrane. In the case where the support layer 2 is a multiple layer membrane, the support layer 2 may be a laminate of a nonporous membrane and a porous membrane. It should be noted, however, that the support layer 2 may be a layer that allows the active ingredient to pass therethrough unless it inhibits the release of the active ingredient from the release control layer 3. The support layer 2 may be a membrane that functions as the release control layer 3.

Examples of the nonporous membrane include a resin film containing polyester (polyethylene terephthalate (PET), for example), nylon, polyvinyl chloride, polyethylene, polypropylene, an ethylene-vinyl acetate copolymer, polytetrafluoroethylene, and an ionomer resin or the like, and a metal foil. Examples of the porous membrane include paper, a woven fabric, a nonwoven fabric (a polyester nonwoven fabric, for example), and a porous membrane obtained by punching the above-mentioned resin film or metal foil. From the viewpoint of flexibility of the support layer 2, the porous membrane is preferably paper, a woven fabric, or a nonwoven fabric.

The thickness of the support layer 2 is not particularly limited. It is preferably 2 to 200 µm, and more preferably 10 to 50 µm. Such a thickness allows the support layer 2 to have a sufficient flexibility and to support the release control layer 3 sufficiently.

### (Release control layer)

Hereinafter, first to third embodiments of the release control layer will be described. The release control layer may have, in a proper combination, the characteristics described in each of the embodiments.

### (First embodiment of release control layer)

In the present embodiment, at least one of the following conditions holds: i) the release control layer 3 includes a microporous membrane having an average pore diameter of 0.01 µm or less; and ii) the release control layer 3 is a nanofiltration membrane (an NF membrane) or a reverse osmotic membrane (an RO membrane). Thereby, the release control layer 3 can control appropriately the quantity of the active ingredient passing therethrough. The average pore diameter in the condition i) may be 0.1 nm or more, and it may be 0.001 µm or more.

First, the condition i) mentioned above will be described. When the condition i) holds, the release control layer 3 is an ultrafiltration membrane (a UF membrane), for example. The UF membrane generally has a microporous membrane with an average pore diameter in the range of 0.001 µm to 0.01 µm, and a porous support body supporting this microporous membrane. In the release control layer 3, the microporous membrane constitutes, for example, the principal surface 31 of the release control layer 3. The average pore diameter of the microporous membrane can be specified by the following method. First, the microporous membrane is observed with a scanning electron microscope. In an electron microscope image thus obtained, the pore diameters (the diameters of pores) of at least 50 pores or, if possible, 100 pores are calculated, and the average of the calculated values is determined as the average pore diameter of the microporous membrane. In the present description, the pore diameter means the diameter of a circle having the same area as that of a pore, in the electron microscope image, calculated by image processing.

When the molecular weight of the compound having the largest molecular weight among those of the compounds contained in the active ingredient retained in the retention chamber 6 is defined as Mw, an average pore diameter R (µm) of the microporous membrane satisfies R ≤ Mw/15000, for example, and it preferably satisfies R ≤ Mw/35000. The average pore diameter R (µm) may satisfy R ≥ Mw/350000. When the average pore diameter R (µm) satisfies R ≤ Mw/15000, the average pore diameter R may be a value greater than 0.01 µm in some cases.

The density of pores in a surface of the microporous membrane is not particularly limited. It is 10 to 100000 pores/µm², for example, and it is preferably 100 to 10000 pores/µm². The thickness of the microporous membrane is not particularly limited. It is 0.001 to 2 µm, for example, and it is preferably 0.005 to 1 µm.

The material of the microporous membrane is not particularly limited, and it is possible to use a polymer material such as cellulose ester (cellulose acetate, for example), polyethylene, polypropylene, polysulfone, polyvinylidene fluoride, and polyethersulfone.

The porous support body is not particularly limited as long as it can support the microporous membrane. The porous support body is, for example, a nonwoven fabric, or a material obtained by forming a porous layer on a nonwoven fabric. The porous layer has an average pore diameter that is greater than 0.01 µm and equal to or less than 0.4 µm. The material of the porous layer is, for example: polyaryl ether sulfone such as polysulfone and polyethersulfone; polyimide; and polyvinylidene fluoride. From a chemical, mechanical, and thermal stability point of view, the material of the porous layer is preferably polysulfone or polyaryl ether sulfone. The porous support body may be a self-supporting support body composed of a thermosetting resin such as an epoxy resin. In this case, the porous support body has an average pore diameter that is greater than 0.01 µm and equal to and less than 0.4 µm, for example. The thickness of the porous support body is not particularly limited. It is 10 to 200 µm, for example, and it is preferably 20 to 75 µm.

Next, the condition ii) mentioned above will be described. In the above-mentioned condition ii), the release control layer 3 is an NF membrane or an RO membrane. The release control layer 3 is preferably an NF membrane. In the present description, the NF membrane means a membrane that removes sodium chloride at a removal percentage of 5% or more and less than 93% when filtering a test liquid with a sodium chloride concentration of 2000 mg/L under an operating pressure of 1.5 MPa. The RO membrane means a membrane that removes sodium chloride at a removal percentage of 93% or more when filtering a test liquid with a sodium chloride concentration of 2000 mg/L under an operating pressure of 1.5 MPa.

Each of the NF membrane and the RO membrane usually has a dense layer, and a porous support body supporting the dense layer. In the release control layer 3, the dense layer constitutes, for example, the principal surface 31 of the release control layer 3. The thickness of the dense layer is not particularly limited. It is 0.001 to 2 µm, for example, and it is preferably 0.005 to 1 µm. As the porous support body, the above-mentioned materials can be used.

The material of the dense layer is not particularly limited, and there can be used, for example, a polymer material such as modified polyethersulfone, cellulose ester (cellulose acetate, for example), polyamide (aromatic polyamide, for example), polyester, polyimide, vinyl polymer, polyethersulfone, and an ethylene-vinyl alcohol copolymer. The dense layer is preferably made of modified polyethersulfone. In this case, a surface of the dense layer has a high charge density. Thus, the dense layer is swollen with a polar solvent easily. In the case where the liquid 7 contains a polar solvent, applying a pressure to a principal surface of the dense layer swollen with the polar solvent makes it possible for the active ingredient to pass through the dense layer easily together with the polar solvent.

The thickness of the release control layer 3 is not particularly limited. It is preferably 10 to 200 µm, and more preferably 20 to 75 µm. The area of the principal surface 31 of the release control layer 3 is not particularly limited. It is 1 to 1000 cm², for example, and it is preferably 5 to 500 cm².

### (Second embodiment of release control layer)

In the present embodiment, the molecular weight cutoff of the release control layer 3 is 20000 or less, for example, and it is preferably 10000 or less, and more preferably 3000 or less. The molecular weight cutoff of the release control layer 3 may be 200 to 20000, and it may be 200 to 1000. The molecular weight cutoff of the release control layer 3 is determined by the configuration of the microporous membrane, that of the dense layer, etc. The molecular weight cutoff of the release control layer 3 can be specified by a known method. An example of the method that specifies the molecular weight cutoff of the release control layer 3 is as follows. First, a plurality of polyethylene glycols that respectively have average molecular weights different from each other and that each exhibit monodisperse molecular weight distribution. An aqueous solution containing one of the polyethylene glycols at a concentration of 5000 ppm is supplied to a membrane surface of the release control layer 3 under the conditions in which the temperature is 25°C and the pressure is 4 kg/cm². Thereby, the removal rate for the polyethylene glycol can be measured. The removal rate(s) for the other polyethylene glycol(s) is measured in the same manner. A fractionation curve showing a relationship between the obtained removal rates and the average molecular weights of the polyethylene glycols is created. Based on the fractionation curve, the average molecular weight of the polyethylene glycol that results in a removal rate of 90% is specified. The specified average molecular weight can be determined as the molecular weight cutoff of the release control layer 3.

The molecular weight cutoff of the release control layer 3 may be, for example, 2 to 20 times, and may be 4 to 10 times the molecular weight Mw of the compound having the largest molecular weight among those of the compounds contained in the active ingredient retained in the retention chamber 6. When the molecular weight cutoff of the release control layer 3 is 2 to 20 times the molecular weight Mw, the molecular weight cutoff may be a value greater than 20000 in some cases.

### (Third embodiment of release control layer)

In the present embodiment, the release control layer 3 controls appropriately a releasing speed at which the active ingredient is released from the retention part 1. In a state where no pressure is applied to the retention part 1 from the outside, the releasing speed at which the active ingredient is released per area of 1 cm² of the principal surface of the release control layer 3 is less than 0.39 µg/hour, for example, and it is preferably less than 0.30 µg/hour, more preferably less than 0.20 µg/hour, still more preferably less than 0.10 µg/hour, and particularly preferably 0 µg/hour. As stated herein, a "pressure" means a pressure applied from the outside of the retention part 1, and it excludes a pressure caused by the weight of the retention part 1 itself including the liquid 7. In a state where a pressure is applied to the retention part 1 from the outside in such a manner as to transmit a pressure of 0.15 MPa to the principal surface of the release control layer 3, the releasing speed at which the active ingredient is released per area of 1 cm² of the principal surface of the release control layer 3 is 0.39 µg/hour or more, for example, and it is preferably 1 µg/hour or more, more preferably 5 µg/hour or more, still more preferably 10 µg/hour or more, and particularly preferably 40 µg/hour or more.

The releasing speed at which the active ingredient is released per area of 1 cm² of the principal surface of the release control layer 3 is not limited to the above-mentioned ranges. For example, in a state where a pressure is applied to the retention part 1 from the outside in such a manner as to transmit a pressure of 1.0 MPa to the principal surface of the release control layer 3, the releasing speed at which the active ingredient is released per area of 1 cm² of the principal surface of the release control layer 3 may be 0.39 µg/hour or more, for example, and it may be 1 µg/hour or more, 5 µg/hour or more, 10 µg/hour or more, and 40 µg/hour or more.

The releasing speed at which the active ingredient is released per area of 1 cm² of the principal surface of the release control layer 3 can be measured with a measuring device 80 shown in FIG. 2. The measuring device 80 is, for example, a polypropylene filter holder, PPH-47, available from WINTEC, that includes a holder 81, a fixing member 82, and a pressure-applying member 83. The holder 81 has a protruding portion 81a to dispose thereon a membrane 3a (a release control membrane 3a) corresponding to the release control layer 3, and an opening 81b for releasing the liquid 7 that has passed through the membrane 3a. The pressure-applying member 83 is disposed above the membrane 3a. The pressure-applying member 83 has an opening 83a for applying a pressure to a principal surface of the membrane 3a. The fixing member 82 can fix the membrane 3a and the pressure-applying member 83 by being screwed with the holder 81. The fixing member 82 has an opening 82a for exposing the opening 83a of the pressure-applying member 83 to the outside.

The releasing speed at which the active ingredient is released can be measured by the following method. First, the release control membrane 3a is disposed on the protruding portion 81a of the holder 81, and furthermore, the liquid 7 is poured on the membrane 3a. Next, the pressure-applying member 83 is disposed on the liquid 7. The fixing member 82 is screwed with the holder 81 to fix the membrane 3a and the pressure-applying member 83. Subsequently, an inert gas, such as nitrogen, is introduced from the opening 83a of the pressure-applying member 83 in order to apply a pressure to the principal surface of the membrane 3a. The measuring device 80 is left at rest for a certain period of time (3 hours, for example) in a state where a certain pressure (0.15 MPa, for example) is applied to the principal surface of the membrane 3a. The liquid 7 released from the opening 81b is collected in a container 90, and the weight of the released liquid 7 and the concentration of the active ingredient in the liquid 7 are measured. Based on the weight of the released liquid 7 and the concentration of the active ingredient in the liquid 7, it is possible to calculate the releasing speed at which the active ingredient is released per area of 1 cm² of the principal surface of the release control membrane 3a, that is, the release control layer 3.

### (Adhesive layer)

As the adhesive layer 4, an adhesive layer that is used for known transdermal absorption preparations can be used. The adhesive layer 4 contains adhesive polymer, for example. The content of the adhesive polymer in the adhesive layer 4 is 50 weight% or more, for example. Examples of the adhesive polymer include: acrylic polymer such as (meth)acrylic acid ester polymer; rubber polymer such as a styrene-diene-styrene block copolymer (a styrene-isoprene-styrene block copolymer (SBS), a styrene-butadiene-styrene block copolymer (SIS)), polyisoprene, polyisobutylene, and polybutadiene; silicone polymer such as silicone rubber, dimethylsiloxane base, and diphenylsiloxane base; viny-ether polymer such as polyvinyl methyl ether, polyvinyl ethyl ether, and polyvinyl isobutyl ether; vinyl ester polymer such as a vinyl acetate-ethylene copolymer; and ester polymer composed of a carboxylic acid component such as dimethyl terephthalate, dimethyl isophthalate, and dimethyl phthalate, and a polyhydric alcohol component such as ethylene glycol. The adhesive polymer may have a crosslinked structure.

As the acrylic polymer, a copolymer of (meth)acrylic acid alkyl ester and a functional monomer is preferable. The acrylic polymer contains, as a main component, a structural unit derived from (meth)acrylic acid alkyl ester. In the present description, the "main component" means a structural unit that is contained in a polymer at a percentage of 50% or more based on weight. The acrylic polymer contains 50 to 99 weight% of, preferably 60 to 95 weight% of the structural unit derived from (meth)acrylic acid alkyl ester.

As an alkyl group of the (meth)acrylic acid alkyl ester, there can be mentioned, for example, a linear or branched alkyl group (such as a butyl group, a pentyl group, a hexyl group, a heptyl group, an octyl group, a 2-ethyl hexyl group, a nonyl group, a decyl group, an undecyl group, a dodecyl group, and a tridecyl group) having 4 to 13 carbon atoms. The (meth)acrylic acid alkyl ester is preferably 2-ethylhexyl acrylate. The acrylic polymer may contain a structural unit derived from one kind of, or two or more kinds of (meth)acrylic acid alkyl esters.

The functional monomer is a compound having, in its molecule, at least one unsaturated double bond involved in a copolymerization reaction while having a functional group in its side chain. Examples of the functional monomer include: a monomer containing a carboxyl group, such as (meth)acrylic acid, itaconic acid, maleic acid, and maleic anhydride; a monomer containing a hydroxyl group, such as (meth)acrylic acid hydroxyethyl ester and (meth)acrylic acid hydroxypropyl ester; a monomer containing a sulfoxyl group, such as styrene sulfonic acid, allyl sulfonic acid, sulfopropyl(meth)acrylate, (meth)acryloyloxy naphthalenesulfonic acid, and acrylamide methylpropanesulfonic acid; a monomer containing an amino group, such as (meth)acrylic acid aminoethyl ester, (meth)acrylic acid dimethylaminoethyl ester, and (meth)acrylic acid tert-butylaminoethyl ester; a monomer containing an amide group, such as (meth)acrylamide, dimethyl(meth)acrylamide, N-methylol(meth)acrylamide, N-methylolpropane(meth)acrylamide, and N-vinylacetamide; and a monomer containing an alkoxyl group, such as (meth)acrylic acid methoxyethyl ester, (meth)acrylic acid ethoxyethyl ester, (meth)acrylic acid methoxyethylene glycol ester, (meth)acrylic acid methoxydiethylene glycol ester, (meth)acrylic acid methoxypolyethylene glycol ester, (meth)acrylic acid methoxypolyprene glycol ester, and (meth)acrylic acid tetrahydrofuryl ester. The acrylic polymer may contain a structural unit derived from one kind of, or two or more kinds of functional monomers. The functional monomers each are preferably a monomer containing a carboxyl group, and particularly preferably (meth)acrylic acid, from the viewpoints of pressure-sensitive adhesiveness of the adhesive layer 4, cohesiveness of the adhesive layer 4, and release property of the active ingredient from the adhesive layer 4.

The acrylic polymer may further contain a structural unit derived from another monomer other than the (meth)acrylic acid alkyl ester and the functional monomer. Examples of the other monomer include (meth)acrylonitrile, vinyl acetate, vinyl propionate, N-vinyl-2-pyrrolidone, methylvinyl pyrrolidone, vinylpyridine, vinylpiperidone, vinylpyrimidine, vinylpiperazine, vinylpyrrole, vinylimidazole, vinylcaprolactam, and vinyloxazol. The acrylic polymer may contain a structural unit derived from one kind of, or two or more kinds of the other monomers.

In the acrylic polymer, the percentage of the weight of the structural unit derived from the other monomer(s) with regard to the total value of the weight of the structural unit derived from the (meth)acrylic acid alkyl ester and the weight of the structural unit derived from the functional monomer is preferably 0 to 40 weight%, and more preferably 10 to 30 weight%.

As a specific preferable example of the acrylic polymer, a terpolymer of 2-ethylhexyl acrylate, acrylic acid, and N-vinyl-2-pyrrolidone is preferred from the viewpoints of adhesivity to human skin and easiness of repetitive acts of adhesion and separation. In the terpolymer, the weight ratio of the structural unit derived from the 2-ethylhexyl acrylate, the structural unit derived from the acrylic acid, and the structural unit derived from the N-vinyl-2-pyrrolidone is 40 to 99.8:0.1 to 10:0.1 to 50, for example, and it is preferably 52 to 89:1 to 8:10 to 40.

The rubber polymer preferably contains, as a main component, at least one selected from polyisobutylene, polyisoprene, and a styrene-diene-styrene block copolymer (such as SBS and SIS). As the rubber polymer, a mixture obtained by mixing high-molecular-weight polyisobutylene having a viscosity average molecular weight of 500,000 to 2,100,000 with low-molecular-weight polyisobutylene having a viscosity average molecular weight of 10,000 to 200,000 at a weight ratio of 95:5 to 5:95 is particularly preferable from the viewpoints of high stability of the active ingredient, and capability of having both adhesive and cohesive forces required.

In the case where the adhesive layer 4 contains the rubber polymer, it is preferable that the adhesive layer 4 further contain a tackifier. By containing the tackifier, the adhesive layer 4 has an improved adhesiveness at an ordinary temperature. The tackifier is not particularly limited, and a known tackifier can be used. Examples of the tackifier include a petroleum resin (such as an aromatic petroleum resin and an aliphatic petroleum resin, for example), a terpene resin, a rosin resin, a cumarone indene resin, a styrene resin (such as a styrene resin and poly(α-methyl styrene), for example), and a hydrogenation petroleum resin (such as an aliphatic saturated hydrocarbon resin, for example). From the viewpoint of stability of the active ingredient, it is preferable to use an aliphatic saturated hydrocarbon resin as the tackifier. The adhesive layer 4 may contain one kind of, or two or more kinds of tackifiers. The percentage of the weight of the tackifier with respect to the weight of the rubber polymer is 33 to 300 weight%, for example, and it is preferably 50 to 200 weight%.

The adhesive layer 4 may further contain a plasticizer. The plasticizer can add softness to the adhesive layer 4 by plasticizing the adhesive layer 4. This can reduce a pain developed on skin at the time of separating the structure 10 from the skin, and skin irritation. The content of the plasticizer in the adhesive layer 4 is preferably 1 to 70 weight%, and more preferably 20 to 60 weight%.

Examples of the plasticizer include: oils and fats such as olive oil, castor oil, squalene, and lanolin; organic solvents such as decyl methyl sulfoxide, methyl octyl sulfoxide, dimethyl sulfoxide, dimethyl formamide, dimethyl acetamide, methyl pyrrolidone, and dodecyl pyrrolidone; surfactants such as polyoxyethylene sorbitan fatty acid ester, sorbitan fatty acid ester, and polyoxyethylene fatty acid ester; phthalic esters such as dibutyl phthalate, diheptyl phthalate, and dioctyl phthalate; sebacic acid esters such as diethyl sebacate, dibutyl sebacate, and dioctyl sebacate; hydrocarbons such as liquid paraffin; fatty acid esters such as ethyl oleate, diisopropyl adipate, isopropyl palmitate, octyl palmitate, isopropyl myristate, isotridecyl myristate, ethyl laurate, glycerine fatty acid ester, propylene glycol fatty acid ester, and pyrrolidone carboxylic acid fatty acid ester; and ethoxylated stearyl alcohol. The adhesive layer 4 may contain one kind of, or two or more kinds of plasticizers.

The thickness of the adhesive layer 4 is not particularly limited. It is preferably 20 to 300 µm, more preferably 30 to 300 µm, and particularly preferably 50 to 300 µm. In this case, the adhesive layer 4 has a sufficient adhesive strength, and furthermore, the adhesive layer 4 can be produced easily.

From the viewpoint of adhesivity, the adhesive layer 4 is preferably hydrophobic. The content of water in the adhesive layer 4 is preferably 5 weight% or less, more preferably 2 weight% or less, and particularly preferably 1 weight% or less. The content of water in the adhesive layer 4 can be measured with a Karl Fischer coulometric titration method.

### (Separation film)

As the separation film 5, a separation film used for known transdermal absorption preparations can be used. Examples of the separation film 5 include a laminate of a separation film substrate and a separation layer, and a resin film having a high peelability.

Examples of the separation film substrate include: a resin film such as a polyethylene terephthalate (PET) film, a polyimide film, a polypropylene film, a polyethylene film, a polycarbonate film, and a polyester (excluding PET) film, or a metal vapor deposition film obtained by vapor-depositing a metal on these resin films; papers such as Japanese paper, western paper, kraft paper, glassine paper, and fine paper; a fiber material such as a nonwoven fabric and a cloth; and a metal foil.

The separation layer contains a release agent, for example. Examples of the release agent include: polymer containing a long chain alkyl group; silicone polymer (a silicone release agent); and fluorine polymer (a fluorine release agent). The separation layer may be a resin film having a high peelability.

Examples of the resin film having a high peelability include: a polyolefin film containing one kind of, or two or more kinds of polyethylenes (low density polyethylene and linear low density polyethylene, for example), polypropylenes, and ethylene-α-olefin copolymers (ethylene-propylene copolymers, for example); and a film made of Teflon (registered trademark). The ethylene-α-olefin copolymer is a block copolymer or a random copolymer.

The thickness of the separation film 5 is not particularly limited. It is 200 µm or less, for example, and it is preferably 25 to 100 µm.

### (Retention chamber)

The shape of the retention chamber 6 is not particularly limited. Examples of the shape of the retention chamber 6 in a state where no pressure is applied from the outside include a truncated cone shape, a truncated pyramid shape, a columnar shape, a square pillar shape, a dome shape, a spherical shape, an ellipse shape, a lattice shape, and a dot shape. The maximum capacity of the retention chamber 6 is 1 to 1000 cm³, for example, and it may be 10 to 200 cm³. The structure 10 may have a plurality of the retention chambers 6. The total value of the maximum capacities of the retention chambers 6 may be 1 to 1000 cm³, and it may be 10 to 200 cm³.

### (Active ingredient, and liquid containing active ingredient)

The active ingredient is, for example, a physiologically active, particularly pharmacologically active compound. In the present description, the active ingredient containing a pharmacologically active compound is referred to as a drug in some cases. The liquid 7 contains the active ingredient, and further contains, for example, a diluent that dilutes the active ingredient. In the case where the active ingredient is a liquid, the liquid 7 may be substantially composed of the active ingredient. Examples of the active ingredient include a component contained in drugs such as an antifungal drug, a general anesthetic, a sedative and hypnotic drug, an antiepileptic drug, an antipyretic analgesic antiinflammatory drug, an antivertiginous drug, a psychoneurologic drug, a local anesthetic, a skeletal muscle relaxant, a drug for autonomic nerves, an antispasmodic drug, an antiparkinsonian drug, an antihistaminic drug, a cardiotonic drug, a drug for arrhythmia, a diuretic drug, a hypotensive drug, a vasoconstrictor, a coronary vasodilator, a peripheral vasodilator, a drug for arteriosclerosis, a cardiovascular drug, a respiratory stimulant, an antitussive and expectorant drug, a hormonal drug, an external remedy for purulent diseases, an analgesic, antipruritic, astringent and antiinflammatory drug, a drug for parasitic dermatosis, a hemostatic drug, a drug for gout therapy, a drug for diabetes, an antineoplastic drug, an antibiotic, a chemotherapeutic drug, a narcotic drug, an antischizophrenia drug, an antidepressant drug, and a stop-smoking aid drug. The active ingredient may contain no physiologically active compound. The active ingredient may be a component contained in a cosmetic, a perfume, an antiperspirant, an insect repellent, a deodorizer and the like.

The active ingredient contains, for example, one kind of, or two or more kinds of physiologically active compounds. As the physiologically active compounds, there can be mentioned, for example, terbinafine (with a molecular weight of 327.89) having an antifungal effect and an antibacterial effect, and citral (with a molecular weight of 152.2) having a vasorelaxant effect, an antiinflammatory effect, and an analgesic effect.

The molecular weight Mw of the compound having the largest molecular weight among those of the compounds contained in the active ingredient is not particularly limited as long as the compound passes through the release control layer 3. It is 500 or less, for example, and it is preferably 100 to 400. The content of the active ingredient in the liquid 7 is 0.01 to 100 weight%, for example.

The diluent is not particularly limited as long as it can dilute the active ingredient. The diluent may be a polar solvent. Examples of the diluent include: lower alcohols such as ethanol and isopropanol; polyhydric alcohols such as propylene glycol, ethylene glycol, butylene glycol, glycerol, dipropylene glycol, octanediol, and diethylene glycol monoethyl ether; organic acid such as acetic acid, lactic acid, caproic acid, enanthic acid, caprylic acid, oleic acid, and linolic acid; esters such as ethyl acetate, isopropyl myristate, diethyl adipate, and glycerol monooleate; terpenes such as d-limonene, 1-menthol, and mentha oil; laurocapram (Azone); pyrothiodecane; ureas such as urea and 1,3-diphenylurea; and sulfoxides such as dimethyl sulfoxide. The diluent may contain one kind of, or two or more kinds of these compounds. The diluent preferably contains ethanol or propylene glycol. The content of the diluent in the liquid 7 is 0 to 99.99 weight%, for example.

The liquid 7 may further contain an additive such as a stabilizer, a gelatinizer, and a transdermal absorption promoting agent, if necessary.

### (Method of using structure)

Next, an example of the method of using the structure 10 will be described.

First, the structure 10 is attached to skin. The method of attaching the structure 10 to skin is not particularly limited. The structure 10 may be applied to skin via the adhesive layer 4, or may be wound around skin. As mentioned above, no or almost no active ingredient is released from the retention part 1 in a state where no pressure is applied to the retention part 1 from the outside.

Next, a pressure is applied to the retention part 1 from the outside. The application of a pressure changes the shape of the support layer 2. This reduces the internal capacity of the retention chamber 6. When the liquid 7 is retained in the retention chamber 6, a decrease in the capacity of the retention chamber 6 due to the application of a pressure from the outside increases the pressure inside the retention chamber 6. That is, the pressure to the principal surface of the release control layer 3 is increased. Thereby, the releasing speed at which the active ingredient is released from the retention part 1 is increased. The active ingredient released from the retention part 1 moves to the adhesive layer 4. The active ingredient that has moved to the adhesive layer 4 moves to the skin that is in contact with the adhesive layer 4.

As described above, the structure 10 of the present embodiment can control the releasing speed at which the active ingredient is released, depending on the application of a pressure from the outside. That is, the structure 10 of the present embodiment can stop or reduce, as appropriate, the release of the active ingredient from the structure 10 in a state where the structure 10 is in contact with skin. Thereby, the structure 10 has great convenience.

As the release control layer 3 included in the structure 10 of the present embodiment, a membrane used as a semipermeable membrane is usually used. In conventional applications, semipermeable membranes are used for filtration of a specific component contained in a solution or concentration of a specific component in a solution. In contrast, a membrane corresponding to the release control layer 3 of the structure 10 of the present embodiment can be used as a membrane that controls the releasing speed at which the active ingredient is released. As described herein, the structure 10 of the present embodiment includes the release control layer 3 adopted focusing on the characteristics different from the conventionally known characteristics of the semipermeable membranes.

Devices that release an active ingredient when a pressure is applied are proposed in JP 62(1987)-207456 A and JP 2001-95604 A. For example, JP 62(1987)-207456 A discloses a bandage provided with a pressure response means such as a capsule. In this bandage, a capsule is broken by application of a pressure, so that a fluid retained in the capsule is released. JP 2001-95604 A discloses shoes provided with a liquid-type athlete's foot remedy retained in a microcapsule. In the shoes, the microcapsule is collapsed by application of a pressure, so that the athlete's foot remedy is evaporated. In the devices disclosed in these literatures, however, the active ingredient is released at one time when the capsule is broken. In contrast, in the structure 10 of the present embodiment, the active ingredient is released each time a pressure is applied to the retention part 1 as long as the active ingredient is retained in the retention part 1. Accordingly, the structure 10 of the present embodiment can be used repeatedly.

### EXAMPLES

Hereinafter, the present invention will be described in more detail by way of examples and comparative examples. It should be noted that the present invention is not limited to these examples and comparative examples.

### (Example 1)

The releasing speed at which an active ingredient was released per area of 1 cm² of a principal surface of a release control membrane was measured using the polypropylene filter holder, PPH-47, available from WINTEC, that had the same configuration as that of the measuring device 80 shown in FIG. 2. As the release control membrane, NTR-7410 available from Nitto Denko Corporation was used. The NTR-7410 was an NF membrane including a dense layer made of modified polyethersulfone. It was impossible to specify the average pore diameter of the dense layer of the NTR-7410 with a scanning electron microscope (SEM) (S-4800 available from Hitachi High-Tech Corporation), and thus the average pore diameter was confirmed to be smaller than 0.01 µm. The NTR-7410 had a molecular weight cutoff of 3000. The principal surface of the release control membrane had a circular shape and had a diameter of 47 mm. As the liquid, an ethanol (EtOH) solution containing terbinafine (TBF) as the active ingredient was used. The concentration of the TBF in the ethanol solution was 1 weight%.

First, there was measured the releasing speed at which the active ingredient was released in a state where no pressure was applied to the principal surface of the release control membrane. To be specific, the release control membrane was set in the holder of the measuring device, and 3 mL of the liquid was poured on the membrane. Next, the pressure-applying member was disposed on the liquid. The fixing member was screwed with the holder to fix the release control membrane and the pressure-applying member. The releasing speed at which the active ingredient was released per area of 1 cm² of the principal surface of the release control membrane was calculated based on the weight of the liquid released by leaving the measuring device at rest for three hours in this state as well as on the concentration of the active ingredient in the liquid. In Example 1, the releasing speed at which the active ingredient was released per area of 1 cm² of the principal surface of the release control membrane in a state where no pressure was applied to the principal surface of the release control membrane was 0 µg/hour.

Next, a releasing speed at which the active ingredient was released in a state where a pressure was applied to the principal surface of the release control membrane was measured by carrying out the same procedure as that described above, except that a pressure was applied to the principal surface of the release control membrane by introducing nitrogen from the opening of the pressure-applying member after the pressure-applying member was fixed. In Example 1, there was measured the releasing speed at which the active ingredient was released in a state where a pressure of 0.15 MPa, 0.25 MPa, 0.35 MPa, and 0.50 MPa each was applied to the principal surface of the release control membrane. Table 1 shows the results.

### (Example 2)

The releasing speed at which the active ingredient was released per area of 1 cm² of the principal surface of the release control membrane was measured by the same method as in Example 1, except that a propylene glycol (PG) solution containing TBF as the active ingredient was used as the liquid. Table 1 shows the results.

### (Example 3)

The releasing speed at which the active ingredient was released per area of 1 cm² of the principal surface of the release control membrane was measured by the same method as in Example 1, except that an ethanol solution containing citral as the active ingredient was used as the liquid and the concentration of the citral in the ethanol solution was adjusted to 2.3 weight%. Table 1 shows the results.

### (Example 4)

The releasing speed at which the active ingredient was released per area of 1 cm² of the principal surface of the release control membrane was measured by the same method as in Example 3, except that a propylene glycol solution containing citral as the active ingredient was used as the liquid. Table 1 shows the results.

### (Example 5)

The releasing speed at which the active ingredient was released per area of 1 cm² of the principal surface of the release control membrane was measured by the same method as in Example 3, except that a butylene glycol (BG) solution containing citral as the active ingredient was used as the liquid. Table 1 shows the results.

### (Example 6)

The releasing speed at which the active ingredient was released per area of 1 cm² of the principal surface of the release control membrane was measured by the same method as in Example 3, except that NTR-7430 available from Nitto Denko Corporation was used as the release control membrane. The NTR-7430 was an NF membrane including a dense layer made of modified polyethersulfone. It was impossible to specify the average pore diameter of the dense layer of the NTR-7430 with an SEM, and thus the average pore diameter was confirmed to be smaller than 0.01 µm. The NTR-7430 had a molecular weight cutoff of 2000. Table 1 shows the results.

### (Example 7)

The releasing speed at which the active ingredient was released per area of 1 cm² of the principal surface of the release control membrane was measured by the same method as in Example 1, except that NTR-7450 available from Nitto Denko Corporation was used as the release control membrane, and an ethylene glycol (EG) solution containing TBF as the active ingredient was used as the liquid. The NTR-7450 was an NF membrane including a dense layer made of modified polyethersulfone. It was impossible to specify the average pore diameter of the dense layer of the NTR-7450 with an SEM, and thus the average pore diameter was confirmed to be smaller than 0.01 µm. The NTR-7450 had a molecular weight cutoff of 1000. Table 1 shows the results.

### (Example 8)

The releasing speed at which the active ingredient was released per area of 1 cm² of the principal surface of the release control membrane was measured by the same method as in Example 7, except that a butylene glycol solution containing TBF as the active ingredient was used as the liquid. Table 1 shows the results.

### (Example 9)

The releasing speed at which the active ingredient was released per area of 1 cm² of the principal surface of the release control membrane was measured by the same method as in Example 1, except that NTR-7470 available from Nitto Denko Corporation was used as the release control membrane. The NTR-7470 was an NF membrane including a dense layer made of modified polyethersulfone. It was impossible to specify the average pore diameter of the dense layer of the NTR-7470 with an SEM, and thus the average pore diameter was confirmed to be smaller than 0.01 µm. The NTR-7470 had a molecular weight cutoff of 700. Table 1 shows the results.

### (Example 10)

The releasing speed at which the active ingredient was released per area of 1 cm² of the principal surface of the release control membrane was measured by the same method as in Example 3, except that the NTR-7470 available from Nitto Denko Corporation was used as the release control membrane. Table 1 shows the results.

### (Example 11)

The releasing speed at which the active ingredient was released per area of 1 cm² of the principal surface of the release control membrane was measured by the same method as in Example 3, except that PES-10K available from Nitto Denko Corporation was used as the release control membrane, and a grape seed oil solution containing citral as the active ingredient was used as the liquid. The PES-10K was a UF membrane including a microporous membrane made of polyethersulfone. The microporous membrane of the PES-10K had an average pore diameter of 5 nm. The PES-10K had a molecular weight cutoff of 10000. The grape seed oil is a vegetable oil containing fatty acid such as oleic acid and linolic acid. Table 1 shows the results.

### (Example 12)

The releasing speed at which the active ingredient was released per area of 1 cm² of the principal surface of the release control membrane was measured by the same method as in Example 3, except that PROC10 available from Nitto Denko Corporation was used as the release control membrane. The PROC10 was an RO membrane including a dense layer made of aromatic polyamide. It was impossible to specify the average pore diameter of the dense layer of the PROC10 with an SEM, and thus the average pore diameter was confirmed to be smaller than 0.01 µm. The PROC10 had a molecular weight cutoff of less than 300. Table 1 shows the results.

### (Comparative Example 1)

The releasing speed at which the active ingredient was released per area of 1 cm² of a principal surface of Celgard 2400, available from Polypore International, Inc., was measured by the same method as in Example 1, except that the Celgard 2400 was used instead of the NTR-7410. The Celgard 2400 was a single layer membrane made of polypropylene. The Celgard 2400 had an average pore diameter of 0.028 µm. The Celgard 2400 had a molecular weight cutoff of 60000. Table 1 shows the results.

### (Comparative Example 2)

The releasing speed at which the active ingredient was released per area of 1 cm² of the principal surface of the Celgard 2400, available from Polypore International, Inc., was measured by the same method as in Example 3, except that the Celgard 2400 was used instead of the NTR-7410. Table 1 shows the results.

**[[Table 1]**

| | Release control membrane | Molecular weigh t cutoff | Liquid | Releasing speed at which active ingredient was released (µg/cm² • hour) | | | | |
|---|---|---|---|---|---|---|---|---|
| | | | | 0 MPa | 0.15 MPa | 0.25 MPa | 0.35 MPa | 0.50 MPa |
| Example 1 | NTR-7410 (NF membrane) | 3000 | TBF/EtOH | 0 | 43.40 | 92.91 | 160.46 | 158.93 |
| Example 2 | | | TBF/PG | 0 | 15.90 | 6.55 | - | - |
| Example 3 | | | Citral/EtOH | 0 | 233.81 | 129.90 | - | 311.75 |
| Example 4 | | | Citral/PG | 0.01 | 236.53 | 304.07 | - | - |
| Example 5 | | | Citral/BG | 0 | 11.86 | 7.39 | - | - |
| Example 6 | NTR-7430 (NF membrane) | 2000 | Citral/EtOH | 0.20 | 0.39 | 12.99 | - | 129.90 |
| Example 7 | NTR-7450 (NF membrane) | 1000 | TBF/EG | 0 | 13.81 | 0.98 | - | - |
| Example 8 | | | TBF/BG | 0 | 46.60 | - | - | - |
| Example 9 | NTR-7470 (NF membrane) | 700 | TBF/EtOH | 0.14 | 16.90 | - | - | 61.89 |
| Example 10 | | | Citral/EtOH | 0.29 | 311.75 | - | - | - |
| Example 11 | PES-10K (UF membrane) | 10000 | Citral/Grape Seed Oil | 0.29 | 1.93 | 29.41 | - | - |
| Example 12 | PROC10 (RO membrane) | < 300 | Citral/EtOH | 0 | 0.78 | - | - | - |
| Comparative Example 1 | Celgard 2400 | 60000 | TBF/EtOH | 61.13 | 220.06 | - | - | - |
| Comparative Example 2 | | | Citral/EtOH | 129.90 | 441.65 | - | - | - |

In Examples 1 to 12, the releasing speed at which the active ingredient was released in a state where no pressure was applied to the principal surface of the release control membrane was less than 0.39 (µg/cm² • hour) as shown in Table 1. In contrast, the releasing speed at which the active ingredient was released in a state where a pressure of 0.15 MPa was applied to the principal surface of the release control membrane was 0.39 (µg/cm² • hour) or more in Examples 1 to 12. These results reveal that the release control membranes used in Examples 1 to 12 are suitable as the release control layer of the structure of the present embodiment. It should be noted that in Examples 1 to 12, no difference was observed in the concentration of the active ingredient in the liquid between before and after the liquid passed through the release control membrane.

In Comparative Examples 1 and 2, the releasing speed at which the active ingredient was released significantly exceeded 0.39 (µg/cm² • hour) even in the state where no pressure was applied to the principal surface of the Celgard 2400, contrary to Examples 1 to 12. With the Celgard 2400 used in Comparative Examples 1 and 2, the structure cannot stop or reduce, as appropriate, the release of the active ingredient in a state where the structure is applied on skin.

### INDUSTRIAL APPLICABILITY

The structure of the present embodiment can be used for various applications such as a transdermal absorption preparation and a bandage. Particularly, the structure of the present embodiment is useful as a transdermal absorption preparation to be applied to parts, such as soles, knees, and arms, to which a pressure is applied from the outside.

## Claims

1. A structure comprising a retention part that retains an active ingredient, and a release control layer that controls release of the active ingredient to an outside, wherein
at least one of the following conditions holds:
i) the release control layer includes a microporous membrane having an average pore diameter of 0.01 µm or less; and
ii) the release control layer is a nanofiltration membrane or a reverse osmotic membrane.

2. A structure comprising a retention part that retains an active ingredient, and a release control layer that controls release of the active ingredient to an outside, wherein
the release control layer has a molecular weight cutoff of 20000 or less.

3. A structure comprising a retention part that retains an active ingredient, and a release control layer that controls release of the active ingredient to an outside, wherein
a releasing speed at which the active ingredient is released per area of 1 cm² of a principal surface of the release control layer is less than 0.39 µg/hour in a state where no pressure is applied to the retention part from the outside, and
the releasing speed at which the active ingredient is released per area of 1 cm² of the principal surface of the release control layer is 0.39 µg/hour or more in a state where a pressure is applied to the retention part from the outside in such a manner as to transmit a pressure of 0.15 MPa to the principal surface of the release control layer.

4. The structure according to any one of claims 1 to 3, wherein the release control layer is a nanofiltration membrane.

5. The structure according to claim 4, wherein
the nanofiltration membrane includes a dense layer, and
the dense layer is made of modified polyethersulfone.

6. The structure according to any one of claims 1 to 5, wherein the retention part includes a support layer, and a retention chamber formed between the support layer and the release control layer.

7. The structure according to any one of claims 1 to 6, wherein the active ingredient contains a compound having at least one effect selected from the group consisting of an antifungal effect, an antibacterial effect, an antiinflammatory effect, an analgesic effect, and a vasorelaxant effect.

8. The structure according to any one of claims 1 to 7, further comprising an adhesive layer disposed on a principal surface of the release control layer.
